# EUROPEAN PATENT SPECIFICATION

(11) **EP 4 480 688 B1**
(45) Date of publication and mention of the grant of the patent: **11.11.2026**
(21) Application number: 24211577.2
(22) Date of filing: 09.12.2021
(51) Int. Cl.: A41D 13/11, A62B 23/02, A62B 18/02, A62B 18/08, B29C 45/14, B29C 45/17, B29K 21/00, B29K 105/04, B29L 12/00, B29L 22/00, B29L 31/14, B29L 31/26

(54) **FACE MASK**
GESICHTSMASKE
MASQUE FACIAL

(30) Priority: 09.12.2020 GB 202019406; 16.07.2021 GB 202110291
(43) Date of publication of application: 25.12.2024
(62) Document number(s) of the earlier application(s) in accordance with Art. 76 EPC: 21839044.1 / 4 258 923
(73) Proprietor: Intersurgical AG, 9490 Vaduz (LI)
(72) Inventor: Leary, Matthew, 9490 Vaduz (LI); Bottom, David, 9490 Vaduz (LI); Miller, Andrew, 9490 Vaduz (LI); Payne, Simon, 949 Vaduz (LI)
(74) Representative: Adamson Jones

(56) References cited:
- CN-A- 111 109 715
- CN-A- 111 248 546
- CN-A- 111 838 822
- US-A1- 2012 125 341

## Description

The present invention relates to face masks, to methods of manufacturing face masks, and to face masks produced by the disclosed methods.

Face masks are used for a range of reasons including therapy, and for personal protection (in both medical and non-medical settings).

Some masks are required to filter out particulate substances from the air, ie filter masks. Such masks are typically one of two types. In particular, the face mask may have a mask body that itself functions to filter out particulate substances from the air, and this type of filter mask is typically lightweight and relatively inexpensive, so as to be single-use and disposable. Alternatively, the face mask may have a mask body adapted to receive a filter cartridge. Such a filter cartridge may be replaceable, so that the face mask is reusable.

Filter masks may be worn in work or public environments to protect from dust, smoke and other noxious substances, and to protect from pathogens, such as viruses or bacteria. In a healthcare environment, for example, filter masks are used to prevent the inhalation of pathogens from the environment by preventing the passage of particulates, eg droplets and aerosols, through the mask. Filter masks may also protect others from exhalation of pathogens by the wearer.

Filter masks typically comprise a filter, such as a sheet of textile or nonwoven filter material, and a means for retaining the mask on the wearer's head, for example by loops of elastic which are placed around the ears. In use, the filter covers the user's mouth and nose, or air is otherwise directed through the filter, so that any air inhaled by the wearer passes through the filter. In order to protect others from exhalation of pathogens by the wearer, the filter may be arranged so that any air exhaled by the wearer passes through the filter.

Filter masks worn in work environments, such as by healthcare professionals, must stay securely fitted to the face and be comfortable to wear for a substantial period of time in an environment in which the risk of contamination may be high. Such masks must closely fit the wearer's face, and preferably form a seal against the face so that inhaled and exhaled air cannot pass around the edge of the mask.

Filter masks are commonly single-use products, produced and sold in high volume. For products of this type to be commercially viable, the cost and complexity of manufacture must be kept to a minimum.

In many circumstances it is desirable that the mask seals to the wearer's face, to prevent the ingress or egress of air about the periphery of the mask. Creating an effective seal against the varying contours of the face can be challenging, and cushion seals formed of a resilient material that compresses against the face to form a seal are commonly employed. However, such seals can become dislodged during movement of the wearer's face (eg during speech), and may be prone to lateral displacement across the face.

CN111248546 discloses a replaceable protective mask comprising a soft sealing gasket, the soft sealing gasket being arranged on the inner side of the mask main body to form a containing space with the mask main body as the top and the soft sealing basket as the bottom. CN111838822 discloses a reusable silica gel mask comprising a silica gel mask with hook and loop fasteners located on the side portions of the silica gel mask. US2012/125341 discloses a filtering face-piece respirator comprising a mask body. The mask body includes a support structure, a filtering structure and a face seal element overmoulded onto at least a portion of the perimeter of the support structure of the mask body.

There has now been developed a mask and a method for its production which overcomes or substantially mitigates the above mentioned and/or other problems associated with the prior art.

According to an aspect of the invention, there is provided a filter mask comprising a support frame, a filter and a sealing member, wherein the sealing member is fixed to the support frame and the filter.

The support frame and the filter may together define a mask body, which forms a cavity for the accommodation of the wearer's mouth and nose. The mask body may be curved in at least the transverse plane.

The support frame may be made of a plastics material, such as polypropylene, poly(styrene-butadiene-styrene) (SBS), polycarbonate, plastomers, thermoplastics, thermoplastic elastomers, thermosets or blends or combinations of the foregoing.

The support frame may have an external face and an internal face, the internal face, in use, facing the wearer and the external face, in use, facing away from the wearer.

The support frame may support at least a portion of the filter, eg by preventing distortion of the sheet of filtration material. The support frame may provide rigidity to the mask. The support frame may retain the filter media at a distance from the face, thus preventing the filter media from coming into direct contact with the mouth and/or nose and reducing or preventing degradation and contamination of the filter, and increasing comfort for the wearer.

The support frame may take the form of a rim, for example that matches the shape of the filtering material. The rim may have the form of a closed loop. The rim may define an aperture through which inhaled and/or exhaled gases flow, in use. When the filter is placed onto the support frame, the support frame may extend about the periphery of the filter. The support frame may comprise a shoulder extending from an inner edge of the rim, providing a surface on which the filter can be located, facilitating retention of the filter in the mould prior to injection moulding of the sealing member. The filter may be located on the internal face of the support frame. The periphery of the filter may be sealed against the support frame such that air cannot flow between an edge of the filter and the support frame; inhaled air must pass through the filter.

Alternatively, or additionally to the rim, the support frame may comprise one or more ribs, eg a plurality of ribs, which are configured to abut against a surface of the filter, for providing support to the filter and rigidity to the mask. The one or more ribs may have a shape which corresponds to the shape of a surface of the filter. The one or more ribs may be shaped such that they extend across a surface of the filter. The one or more ribs may extend between two opposing sides of the rim or support frame, and be shaped to abut a surface of the filter. The one or more ribs may have an arcuate shape. Such ribs may provide structure and/or rigidity to the rim or support frame, helping to maintain the shape of the mask and prevent distortion of the filter material.

The external face of the support frame may further comprise a shield which, in a filter mask according to the invention, extends over at least part of a surface of the filter. The shield may be separated from the filter, and may be positioned in front of the filter, such that air may flow between the shield and the filter, eg before the air is drawn through the filter by the wearer during inhalation. There may therefore be a space or void between the shield and the filter. An inlet aperture, through which air may be drawn by a wearer inhaling, may be defined between an edge of the shield and a surface of the filter. The shield may not contact the filter.

The shield may provide a barrier upon which particles in the environment impinge. The shield may therefore reduce the quantity of particles incident on the filter during use, and hence may extend the effective life of the filter and hence of the filter mask. The separation between the shield and the filter enables incoming air to flow through the filter across its entire surface, even where the shield is impermeable or substantially impermeable. The shield may additionally provide reinforcement to the mask, preventing longitudinal collapse of the mask, and keeping it in the correct place relative to the wearer's nose and chin.

The shield may be formed of plastics material. This may form a solid barrier to deflect particulates, and reduce the risk of physical damage to the filter by providing a protective barrier. The shield may form a rigid barrier.

The shield may alternatively be a composite shield formed of two or more components. The composite shield may comprise a sheet of filter material (the shield filter) and a shield frame, the shield filter overlaying and being affixed to the shield frame. The shield frame may be a plastic frame, and may form part of the support frame. The shield frame may comprise a shield rim substantially the same shape as the intended shield and/or the shield filter, the shield rim being affixed to the periphery of the shield filter. Such a composite shield actively absorbs and blocks contaminants, and may reduce the build-up of humidity in the mask, increasing comfort for the wearer. The composite shield may be removably fixed to the mask, such that it can be replaced if it becomes saturated; either the entire composite shield may be removeable and replaceable, or just the sheet of filter material may be replaceable.

In the following paragraphs, unless indicated otherwise references to the "shield" refer both to a shield formed of a plastics material, and to a composite shield formed of a shield frame and shield filter. In addition, unless indicated otherwise references to the "filter" refer to the main filter, that is, the filter retained by the support frame, and references to the "shield filter" refer to a filter forming part of a composite shield.

The surface area of the shield may be at least 50%, at least 60%, at least 70%, or at least 80%, or at least 90%, or at least 95% of the surface area of the filter. The surface area of the shield may be up to 110% of the surface area of the filter, or up to 105%, or up to 100%, or up to 95% of the surface area of the filter. The shield may extend over at least 50%, at least 60%, at least 70%, or at least 80%, or at least 90%, or at least 95%, or up to 100% of the surface area of the filter.

The shield may extend over the entire surface area of the filter, or may extend beyond the periphery of the filter such that the shield overhangs the edges of the filter. Alternatively, the shield may extend over less than 100% of the surface area of the filter, such that a portion of the filter is exposed at the periphery of the shield. Alternatively, or additionally, the shield may have apertures formed therein through which the sheet of filtering material is exposed, which may assist the flow of air and help to evenly distribute the particle load on the filter. The shield may alternatively have no apertures, but form a continuous barrier. Thus, the shield may comprise a continuous surface that extends across a majority of the surface of the filtering material.

The support frame may include a shield that extends over at least part of an outwardly-facing surface of the filter, and at least part of the periphery of the shield being separated from the support frame. One or more inlet apertures may be defined between the shield and the support frame, through which air is drawn on inhalation by the wearer.

The support frame may further comprise one or more seal supporting members. The support frame may comprise one or more seal supporting members located at any position at which the seal requires reinforcement. The support frame may comprise one or more seal supporting members in the chin region, the nasal region and/or the cheek region. In particular, the support frame may comprise one or more seal supporting members in the chin region. The one or more seal supporting members provide additional support to the seal and help to prevent collapse of the seal and improve fit. The one or more seal supporting members may extend outwardly from the support frame, abutting the sealing member, and have a shape corresponding to the shape of the portion of the sealing member to which it abuts. The one or more seal supporting members may have an arcuate shape.

At least one seal supporting member may be located in the chin region, and the chin region of the support frame may comprise at least one downwardly depending seal supporting member. The at least one seal supporting member may comprise a tab extending outwardly from the support frame, and may extend downwardly in a direction substantially perpendicular to the support frame. The at least one seal supporting member may extend to the distal edge of the sealing member, or may only extend across a portion of the depth of the sealing member. The at least one seal supporting member may be bonded to the seal, and/or the seal may be overmoulded to the seal supporting member during the overmoulding process. The seal supporting member provides additional reinforcement in the chin region, helping to prevent collapse of the seal in this region. This helps to improve and maintain the seal.

The support frame is typically formed of a plastics material that retains its shape in use. The support frame may comprise a rim and/or a shield or shield frame and/or one or more connection members and/or one or more seal supporting members. Where multiple components are present the entire support frame may be integrally formed, eg by injection moulding, or the support frame may be formed in two or more pieces which are connected together eg by gluing, welding or mechanical fastening. Thus, where the support frame comprises a rim and a shield or shield frame, the rim and shield or shield frame may be integrally formed in one injection moulding step, or the rim and the shield or shield frame may be formed separately before being bonded together either chemically or mechanically.

The filter may be formed from a filter substrate that is sufficiently dense to prevent the passage of a majority of airborne particles, such as dust particles, droplets and aerosols. The mask of the present invention may filter out at least 80%, or at least 85%, or at least 90%, or at least 95% or at least 99% of airborne particles.

In countries including the UK and those in the European Union, the FFP standards specify requirements for filtering masks as respiratory protective devices. The EN 149 standard defines three classes of filter efficiency, namely FFP1, FFP2 and FFP3. The mask of the present invention may be a FFP3 mask, for example, which under the EN 149 standard filters out at least 99% of airborne particles at 95 L/minute airflow, and with a total inward leakage of less than 2%. The mask of the present invention may be a FFP2 mask, which filters out at least 94% of airborne particles at 95 L/minute, and with a total inward leakage of less than 8%. In the USA, the U.S. National Institute for Occupational Safety and Health (NIOSH) provides an N95 classification of air filtration, and the mask of the present invention may be an N95 mask, meaning that it filters at least 95% of airborne particles.

Materials suitable for use as the filter substrate are known in the art, and any suitable material may be used for the sheet of filtering material in the present invention. For example, the filter may be a nonwoven, electrostatic, meltblown, spunbond, textile, nanofiber or nanoweb material, or be formed of a glass fibre media. The filter may comprise a plurality of layers to enhance its performance and increase comfort for the wearer. Thus, the filter may comprise one or more layers selected from nonwoven, electrostatic, meltblown, spunbond, textile, nanoweb and/or nanofiber material. For example, the filter may comprise layers of nonwoven, electrostatic and meltblown material, together with a textile layer to increase comfort for the wearer and provide structure to the filter. The filter may be in any appropriate configuration, for example the surface of the filter may be smooth, eg it may be flat or planar, or the filter may be pleated. The filter (pleated or non-pleated) may have a flat or planar region about its periphery to facilitate the overmoulding process. The filter may further comprise one or more additives to enhance its effectiveness in reducing or preventing the transmission of pathogens, such as antimicrobial agents.

The use of a pleated filter increases the surface area of the filter, improving the effectiveness of the filter. Pleating the filter may increase the surface area by 5-50%, or by 5-30%, or by 10-20% compared to an unpleated filter. To accommodate a pleated filter, the shape of the support frame may correspond to the shape of the pleated filter, ensuring a close fit between the two components and preventing leaks. For example, where the filter abuts a shoulder or rim on the support frame, the shoulder or rim may comprise a series of corrugations which correspond to the pleats in the filter such that, when the filter contacts the shoulder or rim, the corrugations nest with the pleats of the filter to ensure a close fit. The filter may be pleated prior to it being located in the mould with the support frame, and overmoulding of the sealing member to fix the sealing member, support frame and filter together. Alternatively, compression of an unpleated (ie a flat or planar) filter between the corrugated rim or shoulder of the support frame and a correspondingly shaped mould may create the pleats in the filter, prior to overmoulding of the sealing member to fix the sealing member, support frame and filter together.

The filter that is located in the mould in the method of the present invention may comprise the filter substrate only, and may be flexible in form. The filter may include no support for the filter substrate until fixed to the support frame and the sealing member.

The filter may be cut to the correct shape from a larger sheet of filter substrate, for example using a die. Where the filter is formed from a plurality of layers, the filter may be cut to size and the edges sealed in a single hot stamp process, in which heat is used to seal the peripheral edges of the filter during the cutting process. The sealed periphery, in use, prevents ingress of moisture or contaminants to the interior of the filter material, between the layers. The size and shape of the filter may correspond to the size and shape of at least a portion of the support frame. For example, the size and shape of the filter may correspond to the size and shape of the rim of the support frame, such that the filter can be positioned on the rim, eg on a shoulder inwardly extending from the rim, such that the support frame extends about the periphery of the filter.

In some embodiments, the filter mask may comprise a single filter. In some embodiments, the filter mask may comprise two, or more filters. The filter mask may comprise a first filter at a first side of the mask, and a second filter at a second side of the mask. Each of the first and second filters may be brought into contact with the support frame, before being fixed to both the support frame and the sealing member by the overmoulding of the sealing member. In this embodiment, the support frame may comprise a housing having first and second apertures, in which the first and second filters may be located. The size and shape of each filter may correspond to the size and shape of the relevant aperture in the support frame, such that the filter can be positioned on the support frame , eg on a shoulder inwardly extending from a periphery of the aperture, such that the support frame extends about the periphery of each filter.

The sealing member may be shaped to fit snugly and to seal against the wearer's face, such that all of the air inhaled by the wearer passes through the filtering material. To increase comfort and sealing effectiveness, the sealing member may be in the form of a cushion. That is, the sealing member may form a resiliently compressible region between the support frame and the wearer's face, increasing comfort and improving the seal.

Additives may be used to increase the effectiveness of the mask at preventing the passage of pathogens. According to a further aspect of the invention there is provided a filter mask comprising a support frame, a filter and a sealing member, wherein the sealing member is fixed to the support frame and the filter, and wherein at least a portion of the filter mask comprises one or more additives selected from antibacterial agents, antimicrobial agents, antiviral agents and antifungal agents.

Any aspect of the support frame and/or seal and/or filter may comprise one or more additives to help increase the effectiveness of the mask in reducing or preventing the spread of bacteria or other pathogens, including but not limited to antibacterial agents, antimicrobial agents, antiviral agents and/or antifungal agents. In particular, an additive containing silver ions (for example in the form of Silver Knight^{™}) may be incorporated into the plastic of the support frame and/or seal. Silver ions have been found to catalyse the deactivation of pathogenic bacteria and prevent their proliferation, reducing microbial growth within and on the surface of the mask.

The sealing member and, therefore, the face mask, may also extend laterally across the wearer's cheeks, such that a seal is formed between the mask seal and the tissue of the cheeks, and the internal cavity of the mask extends over the cheeks.

During speech, the majority of motion in the face is in the jaw, including vertical separation of the nose and chin, while there is a relatively small amount of movement in the cheeks. In addition, the tissue of the cheeks is softer and more compliant than the tissue of either the nose or chin. This means that a lower degree of flexibility is required in the sealing member to achieve an effective seal with the cheek tissue than with other parts of the face. In addition, sealing in a position further away from the mouth and its movement helps to provide stability and maintain a constant seal.

According to a further aspect of the invention, there is provided a face mask comprising a mask body that defines a cavity for accommodating the nose and mouth of a wearer and from which a wearer inhales respiratory gases, in use, and a sealing member depending from the mask body for engagement with the wearer's face, the mask body comprising a nasal portion for accommodating the nose of the wearer, a mouth portion for accommodating the mouth of the wearer, and cheek portions extending laterally across the wearer's cheeks.

The sealing member may extend about the periphery of the mask, such that the sealing member comprises corresponding cheek portions which extend laterally across the wearer's cheeks.

It has been found that, when the mask body extends laterally across the wearer's cheeks, the additional lateral stability provided makes it easier for the user to position the mask correctly, and helps to prevent the mask from slipping to either side during use. This lateral stability, and the effective seal produced, makes this mask body and sealing formation suitable for use in other types of mask, including gas delivery masks, and gas delivery filter masks (eg oxygen or aerosol delivery).

The cheek portions may extend laterally across the wearer's cheeks between the upper edges of the malar bones and the lower edge of the mandible of the wearer. The laterally extended cheek portions of the mask may form the part of the mask having the longest dimension in the horizontal direction (ie widthwise, running from a first side edge of the mask to an opposing side edge of the mask). The distance between a first side edge of the mask body and an opposing side edge of the mask body, measured along the surface of the mask body, may be between 1.2 and 2 times, or between 1.2 and 1.8 times, or between 1.3 and 1.7 times, or between 1.4 and 1.6 times larger than the distance between the top edge of the mask body in the nasal region and the bottom edge of the mask body in the chin region, measured along the surface of the mask body.

The first side edge and second side edge of the mask, located in the cheek regions, may comprise a straight or substantially straight or linear edge. The first and second side edges may not be arcuate.

The cheek portions may each have a first edge that extends from a left- or right-side edge of the nasal portion, such that the first edge of the cheek portion is accommodated over the malar bone of the wearer. The first edge may extend along an arcuate path. The first edge may be a concave exterior edge. The transition between the first edge of the cheek portion and the left- or right-side edge of the nasal portion may be arcuate in form.

The transition between the first edge (between the nasal portion and the cheek portion) and the first and/or second side edge (in the cheek portion) may be angular. The transition between the first edge and the first and/or second side edge may be at an angle of between 70 and 110 degrees, or between 80 and 100 degrees, or about 90 degrees.

The first side edge may connect the first and second edges at a distal end of the cheek portion (eg proximate to the wearer's ear). The second side edge may connect the first and second edges at a distal end of the opposing cheek portion (eg proximate to the wearer's other ear).

The cheek portions may each have a second edge that extends from a left- or right-side edge of the mouth portion, such that the second edge of the cheek portion is accommodated above the lower edge of the mandible of the wearer. The second edge may extend along an arcuate path. The second edge may be a concave exterior edge. The transition between the second edge of the cheek portion and the left- or right-side edge of the mouth portion may be arcuate in form.

The transition between the second edge (between the mouth portion and the cheek portion) and the first and/or second side edge (in the cheek portion) may be angular. The transition between the second edge and the first and/or second side edge may be at an angle of between 70 and 110 degrees, or between 80 and 100 degrees, or about 90 degrees.

The tangential angle between a tangent line to the first and/or second edges of each cheek portion and the x-axis may decrease with increasing lateral position. The angle between tangent lines to the first and second edges of each cheek portion may decrease with increasing lateral position. At a distal end of the cheek portion, eg proximate to the wearer's ear, the first and second edges may be substantially parallel. At a distal end of the cheek portion, the first edge may be substantially horizonal and/or linear (extending, in use, in a direction widthwise across the wearer's face), while the second edge is arcuate or substantially arcuate. The first and/or second side edge of each of the cheek portions may be generally linear, and may be aligned with the longitudinal axis of the mask (ie the y-axis). A distal region of each cheek portion may be generally rectangular in shape. This may increase the surface area of the mask body that is covering the wearer's cheeks.

The mask body may extend a greater distance in a transverse direction across the face of the wearer, relative to the distance the mask body extends in the longitudinal direction across the face of the wearer. The greater distance may be relative to the surface of the face of the wearer, ie the distances may be dimensions along the surface of the face, as opposed to linear dimensions.

The mask body may extend a greater distance in a transverse direction, relative to the distance the mask body extends in a longitudinal direction. The greater distance may be relative to the surface of the mask, ie the distances may be dimensions along or parallel to the surface of the mask, as opposed to linear dimensions.

The nasal and chin portions of the sealing member may seal against or about the nose and chin, while the cheek portions extend laterally across and seal against the wearer's cheeks. The chin portion may seal against the front of the wearer's chin, or may seal under the chin. The chin portion of the sealing member may not extend under the mandible. The sealing member may not extend about the wearer's eyes and/or forehead.

The depth of the sealing member in the cheek portion may be smaller than the depth of the sealing member in the nasal portion. The depth of the mask body in the cheek portion may be smaller than the depth of the mask body in the nasal portion. Thus, the portion of the internal cavity formed around the cheeks by the cheek portion of the face mask may be shallower than the portion of the internal cavity formed around the nose and mouth by the nasal and mouth portions of the face mask. The portion of the internal cavity formed around the cheeks by the cheek portions of the face mask may have a smaller internal volume than the portion of the internal cavity formed around the nose and mouth by the nose and mouth regions of the face mask.

The sealing member in the cheek region may comprise a first portion and a second portion, the first portion extending rearwardly, in use, from a peripheral edge of the support frame. The first portion may comprise a proximal edge, which is fixed to the support frame, and a distal edge. The second portion may comprise an outwardly depending lip, extending from the distal edge of the first portion. The second portion may define a face contacting surface. The outwardly depending lip of the second portion may extend between 1 and 10mm, or between 2 and 6mm, from the distal edge of the first portion. The thickness of the material forming the first portion may be greater than the thickness of the material forming the second portion. It has been found that the incorporation of an outwardly depending lip in the cheek region helps to form an effective seal in this region, and to retain the seal even during facial movement.

At least a portion of the sealing member in the chin region may comprise a first portion and a second portion, the first portion extending rearwardly, in use, from a peripheral edge of the support frame. The first portion may comprise a proximal edge, which is fixed to the support frame, and a distal edge. The depth of the first portion may vary along its length. The second portion may comprise both an inwardly and an outwardly depending lip, wherein the second portion is fixed to the distal edge of the first portion. The second portion may define a face contacting surface. The outwardly depending lip of the second portion may extend between 1 and 20mm, or between 5 and 20mm, from the distal edge of the first portion. The inwardly depending lip of the second portion may extend between 1 and 15mm, or between 5 and 15mm, from the distal edge of the first portion. The thickness of the material forming the first portion may be greater than the thickness of the material forming the second portion.

The portions of the mask body which accommodate the wearer's nose and mouth may have an exterior surface with a generally arcuate shape, eg in the transverse plane, while the portion which accommodates the wearer's cheeks may have an exterior surface that is substantially more planar in shape, eg in the transverse plane. The face mask, and the internal cavity of the face mask, may extend laterally across the wearer's cheeks, such that an edge of the mask is situated, in use, within 5cm, or within 4 cm, or within 3cm, or within 2cm of the wearer's ears. The face mask may extend laterally across the wearer's cheeks, such that an edge or a corner of the mask is situated, in use, on or about the zygomatic bone, or on or about the zygomatic process. Thus, the mask may substantially cover the wearer's cheeks.

The mask body may comprise a side wall extending around at least a portion of its periphery. The side wall may form part of the support frame. The side wall may have a greater depth in the nasal region than in the cheek regions, in order to accommodate the wearer's nose. The side wall may have a greater depth in the nasal region than in the chin or cheek regions. The depth of the side wall in the chin and cheek regions may be substantially the same. The cheek regions may not comprise a side wall.

The depth of the side wall may gradually decrease between the nasal region and the cheek region, such that the region of the sidewall having the greatest depth is in the nasal region and a region of the sidewall having the smallest depth is in the cheek region. The depth of the sidewall may be substantially constant between the chin region and the cheek regions. Where present, one or more seal supporting members may extend outwardly from the side wall, eg a seal supporting member may extend outwardly from a central point in the chin region.

A first region of the sidewall may extend between the nasal region and a cheek region. The first region of the sidewall may be generally arcuate in shape and may extend rearwardly from the rim of the support frame (ie towards the wearer's face, in use). At least a portion of the first region of the sidewall may extend rearwardly from the rim of the support frame at an obtuse angle relative to the front face of the mask. The portion of the first region adjacent the nasal region may extend outwardly at an obtuse angle relative to the front face of the face mask. At least a portion of the first region of the sidewall may extend rearwardly from the rim of the support frame at a right angle relative to the front face of the mask, that is, in a direction perpendicular to the front face of the face mask. The portion of the first region adjacent the cheek region may extend rearwardly from the rim at a right angle relative to the front face of the mask.

A second region of the sidewall may extend between the chin region and a cheek region. The second region of the sidewall may be generally arcuate in shape.

Where the face mask is a filter mask, the mask body may be defined by the support frame and the filter. The sealing member may extend about the periphery of the support frame, eg about the periphery of the rim. The shape of at least a portion of the support frame and of the filter may correspond to the shape of the proximal edge of the sealing member. The support frame and the filter may extend laterally across the wearer's cheeks. The portions of the mask body which extends across the wearer's cheeks may form a shallower cavity between the mask and the wearer's face than the portion of the mask which extends over the wearer's nose and mouth.

The extension of the mask over the cheeks increases the surface area of the sheet of filtering material, thus increasing the volume of air which can pass through the filter. At the same time, the shallower cavity in this region of the mask reduces the dead space within the mask.

Embodiments in which the cheek portions extend laterally across the wearer's cheeks may be advantageous in therapy masks, including but not limited to nebuliser masks, sleep apnoea masks, CPAP masks, oxygen delivery masks, NIV masks and aerosol masks. The breadth of the mask in the cheek regions provides a more stable fit on the wearer's face, making it less likely to move or become dislodged during use.

Masks according to this embodiment of the invention may be particularly advantageous in situations where the user moves considerably, such as for the treatment of sleep apnoea, and for use in CPAP and non-invasive ventilation. The wearer in such situations is typically a patient who may move and roll, and the low profile of the mask and increased stability provided by the lateral extension of the cheek portions allows the wearer to move more easily, even while asleep, without dislodging the mask. In addition, for elderly patients or other patient groups who may have lost some teeth, the lateral extension of the seal over the cheeks enables a good seal to be obtained with less force in this area. The seal may be overmoulded onto the mask body, or may be manufactured as a separate component, which is attached to the mask body. Appropriate materials for the production of the mask body and seal, and their suitability for overmoulding, would be readily apparent to the person skilled in the relevant technical field (eg materials science). The mask may not include a filter. The mask may include a filter, which may provide controlled leakage of air at a particular pressure. Typically, controlled leaks in masks can cause noise which is irritating to the wearer, and the use of a filter or other permeable material, eg a textile material, to provide a controlled leak would reduce or prevent noise.

Face masks according to this embodiment of the invention may additionally comprise a connector for connection to a nebuliser. As described above, the cheek portions may extend laterally between the upper edges of the malar bones and the lower edge of the mandible of the wearer. A nebuliser is a bulky piece of apparatus, and the weight of the nebuliser can cause displacement of a mask on the face. Lateral extension of the cheek portions provides stability, reducing or preventing displacement of the mask and improving the seal. The nebuliser mask may further comprise at least one filter. The nebuliser mask may comprise one filter, or two filters. Where the nebuliser mask comprises two filters, it may comprise a first filter located at a first side of the mask and a second filter located at a second side of the mask, with the connector for connection to a nebuliser located between the two filters. The support frame may comprise a housing having first and second apertures for the accommodation of the two filters, and an integrally formed connector for connection to a nebuliser.

As the effectiveness of the mask increases with improvement in fit (and reduction in leaks), it is useful to have a mask that may accommodate faces of different sizes, and which remains effective even where the wearer is moving and carrying out an active role, such as in a filter mask for use by a healthcare professional.

According to a further aspect of the invention, there is provided a filter mask comprising a support frame and a sheet of filtering material, the support frame and the sheet of filtering material defining a mask body adapted to provide a cavity in use about the mouth and nose of a wearer, the filter mask includes a sealing member depending from at least a portion of a peripheral edge of the mask body, the sealing member comprising both an inwardly and outwardly depending lip portion relative to the peripheral edge of the mask body.

The sealing member may extend around the entire peripheral edge of the mask body. The portion of the peripheral edge of the support frame about which the inwardly and outwardly depending lip portions extend may comprise a chin portion. The inwardly depending lip portion may extend around a majority of the peripheral edge of the support frame. The inwardly depending lip portion may extend around only a part of the peripheral edge of the support frame. The portion of the peripheral edge of the support frame about which the inwardly depending lip portions extend may comprise a chin portion, and/or may comprise a nasal portion. The portion of the peripheral edge of the support frame about which the inwardly depending lip portions extend may not comprise a cheek portion, to facilitate removal of the seal from the mould. The portion of the peripheral edge of the support frame about which the inwardly depending lip portions extend may comprise a cheek portion, to provide an improved seal. The outwardly depending lip portion may extend around a majority of the peripheral edge of the support frame. The outwardly depending lip portion may extend around only a part of the peripheral edge of the support frame, eg the chin and cheek regions. The nasal portion of the sealing member may not comprise an outwardly depending lip portion.

It has been found that the utility of the above described features extends beyond their use in filter masks, and that a seal having the above described features may be applied to any face mask in which an effective seal is required.

Thus, there is provided a face mask comprising a sealing member depending from at least a portion of a peripheral edge of the mask body, the sealing member comprising a chin region, a nasal region, and cheek regions intermediate the chin and nasal regions, and the sealing member comprising both an inwardly and outwardly depending lip portion relative to the peripheral edge of the mask body, wherein the inwardly depending lip portion extends about the chin and nasal regions of the sealing member, and the outwardly depending lip portion extends about the chin and cheek regions of the sealing member.

It is important to form a good seal against the face, whilst not driving up the cost and complexity of manufacture. It has been found that, while an inwardly depending lip portion provides a good seal against the face, it can hinder manufacture by increasing the difficulty of removing the seal from the mould. By providing an inwardly depending lip portion in the chin and nasal regions, which have the greatest variation in shape/size, the seal is improved. The cheeks are typically flatter and formed of softer tissue and forming a good seal against the cheeks is therefore easier. Thus, only an outwardly depending lip portion in these areas is sufficient to provide a good seal, whilst facilitating manufacture.

The inwardly depending lip portion may be generally planar in form. The inwardly depending lip portion may take the form of an upstanding wall which follows the contour of the peripheral edge of the body. The inwardly depending lip portion may include discontinuities therein that facilitate deformation of the inwardly depending lip portion.

At least a portion of the outwardly depending lip portion may have a contact surface that is concave in shape. The outwardly depending lip portion may be shaped so as to provide a sill or shelf formation beneath the wearer's mandible. The outwardly depending lip portion may take the form of an upstanding wall which follows the contour of the peripheral edge of the body. At least a portion of the outwardly depending lip portion may be planar, and about the wearer's face, eg in the chin region and/or in the cheek region. A proximal portion of the outwardly depending lip portion may engage the chin of the wearer, while a distal portion curves and extends under the chin of the wearer. The outwardly depending lip formation may be shaped in the form of a chin cup. The chin cup may, in use, accommodate at least a portion of the chin of the wearer. The outwardly depending lip portion may be shaped so as to provide both a front lip portion and also an underside lip portion.

The inwardly depending lip portion may contact a lower portion of the wearer's face, eg the wearer's chin, to form a seal. Where a user has a larger face height, the outwardly depending lip portion may pass closely beneath the mandible of a wearer, providing an additional seal. For all wearers, the outwardly depending lip portion may provide an alignment guide for placing the mask on the face, and help to keep the mask in the correct position on the face despite movement of the jaw during use.

The periphery of the support frame in the vicinity of the inner and outer lip portions, in the chin region, may be shaped from a central point or region of the mask by a first radius. The inwardly depending lip portion typically has an inner edge of radius which is smaller than the first radius. The outwardly depending lip portion typically has an outer edge of radius which is larger than the first radius.

The nasal portion of the sealing member may comprise an inwardly depending lip portion, which may present a convex surface for contact with a wearer's face. The nasal portion of the sealing member may include reinforcement formations, eg reinforcement formations on each side of the nose, or reinforcement formations about the entirety of the nasal region. It has been found that reinforcement formations in the nasal region provide stability to the mask, preventing unwanted movement and/or collapse of the seal while maintaining the lateral flexibility required to accommodate different face sizes, contributing to the formation of a reliable seal. This reduces unwanted deformation in this area, or unwanted folding of the seal, improving the fit and making correct fitting of the mask easier.

Such a filter mask may comprise cheek portions which extend laterally across the wearer's cheeks as in the embodiment previously described, and comprising any of the features of that embodiment, the cheek portions extending laterally between the upper edges of the malar bones and the lower edge of the mandible of the wearer. This increases the stability of the mask on the face.

According to a further aspect of the invention there is provided a face mask comprising a support frame and a sealing member, the support frame defining a mask body adapted to provide a cavity in use about the mouth and nose of a wearer, and the sealing member depending from at least a portion of a peripheral edge of the mask body and comprising a nasal portion which, in use, forms a seal against or adjacent to the nose of the wearer, the nasal portion comprising at least one reinforcing member.

The face mask may be a filter mask, or a mask which contains a filter element. The face mask may further comprise a sheet of filtering material. The support frame and sheet of filtering material may together define a mask body adapted to provide a cavity in use about the nose and mouth of a wearer.

The nasal portion of the seal, in use, extends over and about the nose of the wearer, forming a seal in the nasal region.

The at least one reinforcing member may be located on an internal wall of the sealing member. The at least one reinforcing member may be located on an external wall of the sealing member. The at least one reinforcing member may be located both on an internal and an external wall of the sealing member. The at least one reinforcing member may be integrally formed with the sealing member, ie by injection moulding at the same time that the sealing member is formed.

The at least one reinforcing member may comprise one or more ribs positioned on each side of the nasal region of the sealing member such that, in use, one or more ribs are located on each side of the nose. The nasal region may comprise 2, or 3, or 4, or 5, or 6, or 7, or 8 or more ribs positioned on each side of the nasal region of the sealing member. Each of the one or more ribs may extend in a direction substantially perpendicular to the mask body. Each of the one or more ribs may extend from an edge of the sealing member proximal to the mask body, and may extend to an edge of the sealing member distal to the mask body. Each of the one or more ribs may extend across only a central portion of the sealing member, and may not reach one or both of the proximal and distal edges of the sealing member. Each of the one or more ribs may have a consistent depth, or may have a depth which varies along its length, and may have a greater depth in a central region than in an end region. Each of the one or more ribs may have a width of from 0.5 to 3mm, or of from 0.5 to 2mm.

The at least one reinforcing member may comprise a corrugated region and/or a scalloped region and/or at least one ridge.

The at least one reinforcing member may comprise a corrugated region located on each side of the nasal portion. The corrugated region may comprise a series of parallel ridges and grooves, which provide increased rigidity to the affected area of the seal. The corrugations may extend in a direction substantially perpendicular to the mask body. The corrugations may extend from a proximal edge of the sealing member to a distal edge of the sealing member, or may extend across a portion of the side wall of the sealing member, eg a central portion, only, and may not reach one or both of the proximal and distal edges of the sealing member. Each corrugation may have a proximal end and a distal end, with the proximal and/or distal ends of each corrugation having a height relative to the sealing member which gradually decreases to zero. Alternatively, the proximal and/or distal end of each corrugation may form a discrete shoulder. The corrugated region may be located on the internal or external surface of the sealing member. The corrugations may comprise thickened regions of the sealing member. The thickened regions may be located on an internal or external surface of the sealing member. The thickened regions may extend through the wall of the sealing member, or discrete thickened regions may be present on the internal and external surface of the sealing member (which may be either aligned or not aligned with each other), such that they are visible on both the internal and external surfaces of the sealing member. The corrugations may alternatively be formed from the side wall of the sealing member, such that the side wall of the sealing member is shaped to form the corrugations. Such corrugations may be visible on both the internal and external surfaces of the sealing member. The side wall of the sealing member which is shaped to form the corrugations may have a uniform thickness.

The at least one reinforcing member may comprise a scalloped region located on each side of the nasal portion. The scalloped region may comprise a series of undulations in the wall of the sealing member, located on either the internal or external surface of the sealing member. The scallops may comprise thickened regions of the sealing member. The thickened regions may be located on an internal or external surface of the sealing member. The thickened regions may extend through the wall of the sealing member, or discrete thickened regions may be present on the internal and external surfaces of the sealing member (which may be either aligned or not aligned with each other), such that they are visible on both the internal and external surfaces of the sealing member. The scallops may alternatively be formed from the side wall of the sealing member, such that the side wall of the sealing member is shaped to form the scallops. Such scallops may be visible on both the internal and external surfaces of the sealing member. The side wall of the sealing member which is shaped to form the scallops may have a uniform thickness. The scallops may extend in a direction substantially perpendicular to the sealing member. The scallops may extend from a proximal edge of the sealing member to a distal edge of the sealing member, across an internal or external surface of the sealing member or may be formed within the wall of the sealing member such that they are visible on both the internal and external surfaces. Alternatively, the scallops may extend across only a central portion (eg a sidewall) of the sealing member, and may not reach one or both of the proximal and distal edges of the sealing member. Each scallop may have a proximal end and a distal end, with the proximal and/or distal ends of each corrugation having a height relative to the sealing member which gradually decreases to zero. Alternatively, the proximal and/or distal end of each scallop may form a discrete shoulder. The smooth profile of the scalloped regions may provide the required stability while ensuring that the sealing member may be easily removed from the mould.

The at least one reinforcing member may comprise at least one ridge. The at least one ridge may be positioned on the internal surface, external surface, or both internal and external surfaces of the sealing member. The at least one ridge may comprise a thickened portion of the sealing member, extending circumferentially around the nasal region or extending in a direction substantially perpendicular to the sealing member. The at least one ridge may extend from a proximal edge of the sealing member to a distal edge of the sealing member, or may extend across only a central portion of the sealing member, and may not reach one or both of the proximal and distal edges of the sealing member.

It has been found that reinforcing members which extend in a direction substantially perpendicular to the sealing member act to brace the wall of the sealing member, providing a compliant cushion which reduces or prevents collapse of the sealing member in the nasal region, and improving the overall seal formed by the sealing member. It has also been found that such reinforcing members reduce or prevent inversion. Such reinforcing members may extend across a portion of the side wall of the mask, eg a central portion, and/or may extend to or close to the proximal edge of the sealing member, and/or may extend to or close to a distal edge of the sealing member. Where the reinforcing member(s) extend to or close to the distal edge of the sealing member, it has also been found that they may help to fix the relative positions of the proximal and distal portions of the sealing member, reducing or preventing inversion of the seal during fitting and/or wearing, and making correct fitting of the mask simpler. The reinforcing member may additionally extend towards, to, or close to the proximal edge of the sealing member, providing additional support.

The at least one reinforcing member may alternatively be a region of increased thickness in the nasal region of the sealing member. The at least one reinforcing member may comprise a region of increased thickness on either side of the nasal region, ie on either side of the nose, in use. It may alternatively comprise a continuous region of increased thickness extending around the nasal region. The region of increased thickness may comprise a narrow band extending along a central portion of the sealing member, or may comprise substantially the entire sidewall of the sealing member.

It is important that any additional features, such as reinforcing members, do not inhibit or prevent the removal of the seal from the mould. Small and/or narrow raised features may be particularly difficult to remove from the mould, and may require manual removal. Larger structures, such as corrugations, scallops, ridges and thickened regions are typically easier to remove from a mould without damage using ejection pins.

The face mask according to the invention may include means for securing the mask to the wearer, in use. Such means may include one or more cords or straps, for example an elasticated cord or strap, that is fitted around the wearer's head to urge the filter mask against the face of the wearer.

The one or more cords or straps may be formed as a separate component or components, and subsequently be attached to the filter mask either by insertion through a hole or slot and knotting or sealing, or by bonding the cord or strap to the mask. The cord(s) or strap(s) may be bonded to the mask by an adhesive or chemical bond, or by overmoulding. The cord(s) or strap(s) may be worn over the ears of the wearer, or around the back of the wearer's head. This may help prevent a mask from slipping down and/or rising up a wearer's face in use.

Two cords or straps may be provided, to pass around each of the wearer's ears, or to extend around the back of the head, one cord passing above and one below the wearer's ears. A single cord may be provided, which extends about the back of the wearer's head.

Alternatively, a single cord may pass twice across the back of the wearer's head.

The elasticated cord or strap may comprise means for indicating that a desired fit has been achieved. The elasticated cord or strap may comprise indicia which indicate to the user that their desired fit has been achieved. The elasticated cord or strap may comprise a size guide. Once a mask according to the invention has been worn and adjusted to provide the correct fit eg using a fit test, the user may use the indicia to adjust any further mask to the correct size, without the need for a further fit test.

Where the mask is a filter mask, the mask may comprise an aperture in the filter. The aperture may provide a conduit through the filter, or may include or be adapted to receive a further device, such as an exhalation valve. The aperture may bypass the filter material. The aperture in the filter material may be mounted and/or sealed to an aperture in the support frame or to a device mounted to the support frame. Furthermore, the polymeric material that is injected into the mould to form the sealing member may be brought into engagement with the filter and the aperture in the support frame or to the device mounted to the support frame, during injection moulding of the sealing member, in a manner that seals the filter about the aperture or device.

The mask may comprise one or more vents or valves, for example an exhalation valve. An exhalation valve may enable exhaled air to escape from the filter mask without passing through the filter, therefore reducing the heat and humidity retained by the filter, during use. However, the inclusion of an exhalation valve may not be desirable where the filter mask is needed to protect others from pathogens exhaled by the wearer, eg in a healthcare environment. The vent or valve may be located in the shield, or in the rim of the support frame. The valve may be inserted into an aperture in the shield or rim, or may form part of the shield or rim. Where the valve is inserted into or forms part of the shield, it may also extend through the filter material, and the overmoulding step which forms the sealing member may be used to additionally provide a seal between the valve and the filter material and/or between an aperture in the support frame for receiving the valve and the filter material. Where the valve is inserted into or forms part of the rim, a portion of the rim may be shaped to accommodate the valve, eg in the chin region.

Where a valve is included, the injection moulding step for forming the sealing member may also form the valve member. The sealing member and the valve member may therefore be integrally formed. After moulding of the sealing member/exhalation valve, the valve may be inserted either through openings in the filter and shield, or through an opening in the rim. Tension between the valve and the surrounding material (shield, filter or rim) biases it closed. This permits exhaled air to flow from the interior of the mask to the exterior, but prevents the flow of air in the opposite direction.

The support frame, eg the shield, and/or filter may be formed with an aperture to which either a releasable cover or an exhalation valve may be fitted. The overmoulding step which forms the sealing member may be used to additionally provide a seal between the aperture in the support frame for receiving the valve or cover and the filter material. For example, the releasable cover may be secured over the aperture using a snap-fit or screw fit connection, the cover being removed and replaced with an exhalation valve where required. This enables a single mask to be produced which may be used either with or without an exhalation valve. The size of the aperture will be an appropriate size for the attachment of an exhalation valve, and may be 10-50mm in diameter, or 15-40 mm in diameter, or 15-30mm in diameter. Typically, the diameter of the aperture will be approximately 20mm.

The filter mask may further comprise a spigot, which may be used to connect the mask to a tube for introducing gases to, or removing gases from, the mask and/or the wearer. Thus, the filter mask may be used as an oxygen delivery mask or respirator, with the additional protection that inhaled and exhaled air (other than the oxygen being delivered) must pass through the filter. In the same way as with the exhalation valve, the spigot may be releasably attached to an aperture in the shield and, optionally, the filter. The aperture may be sized to accommodate a spigot having a diameter of from 10-50mm in diameter, or 15-40 mm in diameter, or 15-30mm in diameter. Alternatively, the aperture may be sized to accommodate a spigot having a diameter of from 2-10mm in diameter, or 2-8 mm in diameter, or 4-8mm in diameter. The spigot may alternatively be integrally formed with the shield, or with another part of the support frame.

The filter mask may comprise a filter and a gas inlet (eg a spigot), and be suitable for use as a high concentration mask, as an oxygen mask, as a CPAP mask or as a high flow mask. Conventional masks for use in the delivery of high concentrations of oxygen require a reservoir bag to be attached to the inlet, to provide a reservoir of oxygen to be drawn on in the event that the patient breathes in excess of what the oxygen supply can meet. It is believed that the larger internal volume of the mask herein described when compared to conventional masks, particularly where the mask extends laterally across the wearer's cheeks, may remove the need for an additional reservoir bag. In addition, the need to control infection and the spread of contaminants means that the inclusion of a filter is highly desirable, protecting both a patient and those providing their treatment.

The filter mask may comprise a gas inlet (eg a spigot) which extends through or is integrally formed with the support frame or mask body, and a filter. The gas inlet and the filter may be located in different parts of the support frame or mask body. The gas inlet and the filter may be located in first and second regions of the support frame or mask body. The first and second regions may be located on different sides of the wearer's nose and mouth, in use. The filter may be located in a first cheek region of the support frame, and the gas inlet located in a second cheek region of the support frame, such that oxygen flows from the gas inlet to the filter, washing over the wearer's mouth and nose, thus providing a continuous flow of oxygen and helping to draw away exhaled gases.

The support frame or mask body may comprise a housing having a first aperture into which a filter may be received, and a second aperture thorough which a gas inlet may be inserted. Alternatively, the gas inlet may be at least partially integrally formed within the support frame or mask body. It is believed that the reservoir of air held within the filter mask may remove the need for an additional reservoir bag. In addition, the need for an anti-asphyxiation valve is reduced or removed as, in the event of oxygen supply failure, the wearer may inhale ambient air through the filter. The lack of need for a reservoir bag and anti-asphyxiation valve reduces both the complexity and the cost of manufacture.

There is thus provided a face mask, the face mask comprising a mask body that defines a cavity for accommodating the nose and mouth of a wearer and a sealing member depending from the mask body for engagement with the wearer's face, the mask body comprising:
a) a filter and a gas inlet, the filter being located in a first region of the mask body and the gas inlet being located in a second region of the mask body; and
b) a nasal portion for accommodating the nose of the wearer, a mouth portion for accommodating the mouth of the wearer, and cheek portions extending laterally across the wearer's cheeks.

The mask body may be transparent, so that it is possible to see the wearer's face (particularly the lips) through the mask, to enable early identification of problems with breathing.

Alternatively, the filter mask may not comprise a spigot. Such filter masks may be unsuitable for use as an oxygen delivery mask or a respiratory mask.

The filtration mask may additionally or alternatively comprise an access port. For example, the filtration mask may comprise an access port for an endoscope, a nasal cannula, or a high flow nasal cannula, such that an endoscopy may be carried out or cannula worn while still providing the patient and medical practitioner with the protection offered by a filtration mask. Masks having cheek portions that extend laterally over the cheeks may be particularly advantageous for use as endoscopy masks, due to the additional stability provided by the lateral extensions which reduce the need for additional head straps to stabilise the endoscopy mask. Medicaments or oxygen may be delivered in through a high flow nasal cannula, and it is a feature of this type of treatment that aerosolised particles may be generated and released to the environment. Depending on the nature of the patient's illness, such aerosolised particles may be contaminated and pose a risk to those in the vicinity. Wearing a filter mask which allows for access to the airway to deliver a high flow nasal cannula but which contains any generated aerosol thus provides a safer environment for clinicians and other personnel in the vicinity. The access port may comprise an aperture in the filter mask. The aperture may comprise a pair of apertures in the support frame, eg shield, and sheet of filtering material that are in registration with each other, such that a cannula can pass through both apertures. The overmoulding step which forms the sealing member may be used to additionally provide a seal between the aperture in the support frame and the filter material. The apertures in the support frame, shield and/or filtering material may be provided with removable covers, such that they may be covered when not in use.

In use, the wearer of the face mask may exhale expiratory gases into the interior cavity. Thus, a face mask according to the invention, which may be a filter mask, may additionally comprise a sensor to detect the CO₂ concentration in exhaled air, such as that described in GB2019236.5.

In this embodiment the mask body and/or the support frame may be formed of an IR-transparent material. The sensor may be mountable relative to the sensor portion of the face mask. The sensor may have a transmitter of electromagnetic radiation and a receiver of electromagnetic radiation, wherein an enclosing wall of the mask body has a sensor portion including first and second sensor windows, a portion of the interior cavity defined by the enclosing wall being defined between the first and second sensor windows, and the sensor being mounted relative to the sensor portion of the respiratory mask, such that electromagnetic radiation from the transmitter is transmitted, in use, through the first sensor window of the sensor portion of the enclosing wall, through the portion of the interior cavity defined between the first and second sensor windows, through the second sensor window, to the receiver. A face mask having a sensor as described may be advantageous in that the sensor portion being formed in the enclosing wall of the mask body allows the sensor to monitor the patient' s expiratory gases before they leave the mask body, reducing the delay between the gases being exhaled and the measurements being taken. In contrast, using conventional respiratory masks that are connected to a sensor via an exhalation tube or a sampling tube, expiratory gases exhaled by the wearer have to travel from the respiratory mask to the sensor, eg a respiratory monitoring unit, before they can be monitored.

Each of the first and second sensor windows may have an interior surface and an exterior surface. The sensor may be mounted relative to the sensor portion of the respiratory mask with the transmitter disposed adjacent to the exterior surface of the first sensor window and the receiver disposed adjacent to the exterior surface of the second sensor window.

The electromagnetic radiation transmitted by the transmitter and received by the receiver may be infrared radiation. Infrared radiation may refer to radiation having a wavelength between approximately 700 nm and approximately 1 mm.

The first and second sensor windows may be transmissive of the electromagnetic radiation transmitted by the transmitter. For example, the first and second sensor windows may be transmissive of infrared radiation, ie radiation having a wavelength between approximately 700 nm and approximately 1 mm. The first and second sensor windows may also be transmissive to other wavelengths of light, such as visible light. The first and second sensor windows may be more transmissive of the electromagnetic radiation transmitted by the transmitter than the remainder of any of, or any combination of, the mask body, the enclosing wall of the mask body, and the sensor portion.

The first and second sensor windows may be transmissive to at least 50%, 60%, 70%, 80%, 90% or 100% of the electromagnetic radiation transmitted by the transmitter. To obtain an accurate and/or viable reading, the sensor may require that a minimum amount of electromagnetic radiation, or a minimum proportion of the electromagnetic radiation transmitted by the transmitter, is received by the receiver. Hence, the first and second sensor windows may be transmissive of the electromagnetic radiation transmitted by the transmitter such that at least 20%, 30%, 40%, 50%, 60%, 70%, 80% or 90% of the electromagnetic radiation is received at the receiver after passing through the portion of the interior cavity defined between the first and second sensor windows.

The first and second sensor windows may be of a reduced thickness relative to the remainder of any of, or any combination of, the mask body, the enclosing wall of the mask body, and the sensor portion. The first and second sensor windows may have a thickness of up to 0.5mm, up to 0.4mm, up to 0.3 mm, up to 0.2 mm, up to 0.1 mm, or up to 0.05 mm. The thickness of the first and second sensor windows may refer to the distance between the interior surface and the exterior surface of each sensor window.

The first and second sensor windows, eg the interior surface of the first and second sensor windows, may be spaced apart by a distance of between 2mm and 50mm, or between 5mm and 30mm, ie the portion of the interior cavity defined between the first and second sensor windows may have a thickness of between 2mm and 50mm, or between 5mm and 30mm.

The face mask may alternatively be constructed such that the sheet of filtering material is replaceable. This means that it would not be necessary to replace the entire mask after use, but only to replace the filtering material while the mask frame, comprising the support frame and seal, could be reused multiple times. This reduces both cost and waste.

A mask having a replaceable filter according to this embodiment may be produced using the same principles as have been previously described, in a method involving a single overmoulding step which fixes the relative positions of the support frame and seal.

According to a further aspect of the invention, there is provided a method for the manufacture of a filter mask, the filter mask comprising a support frame and a sealing member, the support frame having a housing for receiving a filter, wherein the method comprises:
a) providing the support frame in a mould; and
b) injecting a polymeric material into the mould to form a first sealing member for sealing against the face of a wearer, and a second sealing member for sealing against the filter received by the housing,
wherein the sealing member is brought into engagement with the support frame, during injection moulding of the sealing member, in a manner that fixes the sealing member to the support frame.

According to a further aspect of the invention, there is provided a filter mask manufactured according to the above-described method. There is also provided a filter mask, the filter mask comprising a support frame and a sealing member, the support frame further comprising a housing for receiving a filter, wherein the sealing member is fixed to the support frame. Any features described above or below in relation to filter masks of the invention, including but not limited to a shield, and/or laterally extending cheek portions, and/or one or more reinforcing members , and/or a sealing member comprising inwardly and outwardly depending lip portions, and/or the inclusion of valves or access ports, and/or a plurality of filters, may be present in this embodiment.

The filter may comprise a filter substrate only, which may be releasably attachable to the support frame by any suitable means, eg by clips, or by an interference fit, or by a releasable adhesive, eg an adhesive strip covered by a releasable liner that is removed immediately prior to insertion of the sheet of filtering material.

Alternatively, the filter may comprise a filter substrate retained within a cartridge or other support, which is connectable to the support frame. For example, the cartridge may be connected to the external face of the support frame by one or more snap-fit connectors, or clips, or by a friction fit, or there may be a port provided in the support frame for the accommodation of the cartridge. In this embodiment, and where the mask comprises a shield, the shield may form part of the cartridge or other support such that, when the cartridge is attached to the external face of the mask, the shield is positioned over the external surface of the filter, at least part of the periphery of the shield being separated from the support frame. The shield may alternatively be hingedly attached to the support frame, such that it can be opened to permit insertion of the filter cartridge adjacent to the external face of the support frame, and subsequently closed. The filter cartridge may be retained in place by one or more clips or other attachment members, or the hingedly attached shield may retain the filter cartridge in place.

The filter cartridge may alternatively be connected to the internal face of the support frame by one or more snap-fit connectors, or clips. In this embodiment, the shield (where present) may be present as shown in the drawings and as previously described. For example, the filter mask may comprise a shield which, in use, extends over at least part of an outwardly-facing surface of the filter, at least part of the periphery of the shield being separated from the support frame, and/or the shield being integrally formed with the support frame.

The cartridge may take the form of a rim of plastic, for example injection moulded plastic, which matches the shape and size of the filter substrate and which is fixed about the circumference of the filter substrate. At least a portion of the cartridge may be overmoulded about the circumference of the filter substrate. Alternatively, the entirety of the cartridge may be overmoulded about the periphery of the filter substrate. Thus, the cartridge does not restrict the flow of air through the filter substrate.

Methods for the manufacture of sealing members are known in the art, and any suitable method may be used in combination with the present invention. In particular, the sealing member may be at least partially manufactured using a gas-assisted injection moulding method as described in WO2021/037768 and GB2013108.2. These documents describe the use of gas-assisted injection moulding in the manufacture of a sealing member for a respiratory device. Filtration masks for personal protection require a very effective seal, and it has surprisingly been found that sealing members may be produced by this method which provide the highly effective seal required in a filtration mask.

According to a further aspect of the invention, there is provided a method of manufacturing a sealing member for use in a filter mask, the method comprising the steps of:
(a) providing a mould having a cavity, a polymer injection port and a gas inlet port;
(b) injecting a polymer through the polymer injection port into the cavity of the mould; and
(c) introducing gas through the gas inlet port into the cavity of the mould,
thereby forming a sealing member of the filter mask, wherein at least a portion of the sealing member of the filter mask comprises an internal chamber at least partially bounded by a resiliently deformable enclosing wall formed of the polymer, the enclosing wall including a face-contacting surface, the face-contacting surface having a form that is determined by the cavity of the mould and provides an anatomical fit with a user.

It has been found that, when used to manufacture at least a portion of a sealing member for a filtration mask, the external surface of the resiliently deformable enclosing wall can be shaped to provide an anatomical fit with a wearer or wearer's face, providing the sealing member with an effective seal, whilst the internal chamber bounded by the enclosing wall also enables the sealing member to be urged against the wearer, eg against the wearer's face, in the event that the seal needs to be improved. An effective seal is particularly important in a filtration mask as, if there are leaks, air will circumvent the filter and the mask will lose its efficacy.

Only a portion of the sealing member may be produced according to the above method. It is particularly advantageous for the nasal region of the sealing member of a filtration mask to be produced using the above described method.

It will be understood that any features described in relation to one aspect of the invention may be applied to any other aspect of the invention. Thus, any structural features described in relation to a mask having integral filter, such as a shield, first and second attachment formations, exhalation valve and/or spigot, may be applied to the mask having a replaceable filter described above.

Embodiments of the invention are now illustrated, by way of example only, with reference to the following drawings:
Figure 1 is a perspective view of a support frame of a filtration mask according to the invention;
Figure 2 is a side view of the support frame of Figure 1;
Figure 3 is a perspective view of a filter of a filter mask according to the invention;
Figure 4a is a perspective view of the filter located within the support frame of the first embodiment of a filter mask according to the invention during manufacture;
Figure 4b is the view of Figure 4a, without the filter;

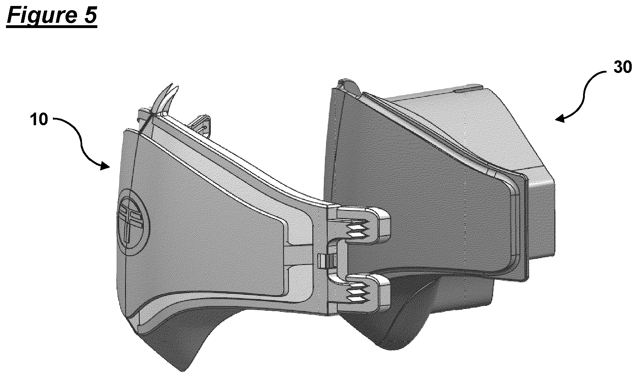

Figure 5 is a perspective view of the support frame and filter of Figure 4 and a core of the mould using during manufacture of the first embodiment of a filter mask according to the invention;
Figure 6 is a side view of a filter mask according to the invention;
Figure 7 is a perspective view of the rear of a filter mask according to the invention;
Figure 8 is a schematic drawing showing the connection between the support frame and the filter material formed by overmoulding of the sealing member;
Figures 9a and 9b are perspective views of the front and rear of a further embodiment of a filter mask according to the invention;
Figures 10a and 10b are perspective views of the front and rear of a further embodiment of a filter mask according to the invention;
Figure 11 is a perspective view of the rear of a filter mask of a further embodiment of the invention;
Figures 12a and 12b show front and rear perspective views of a filter mask according to a further embodiment of the invention;
Figures 13a-d show face masks according to the invention having reinforcing members on each side of the nasal region; and
Figure 14 shows configurations of reinforcing members which may be present in a face mask according to the invention.

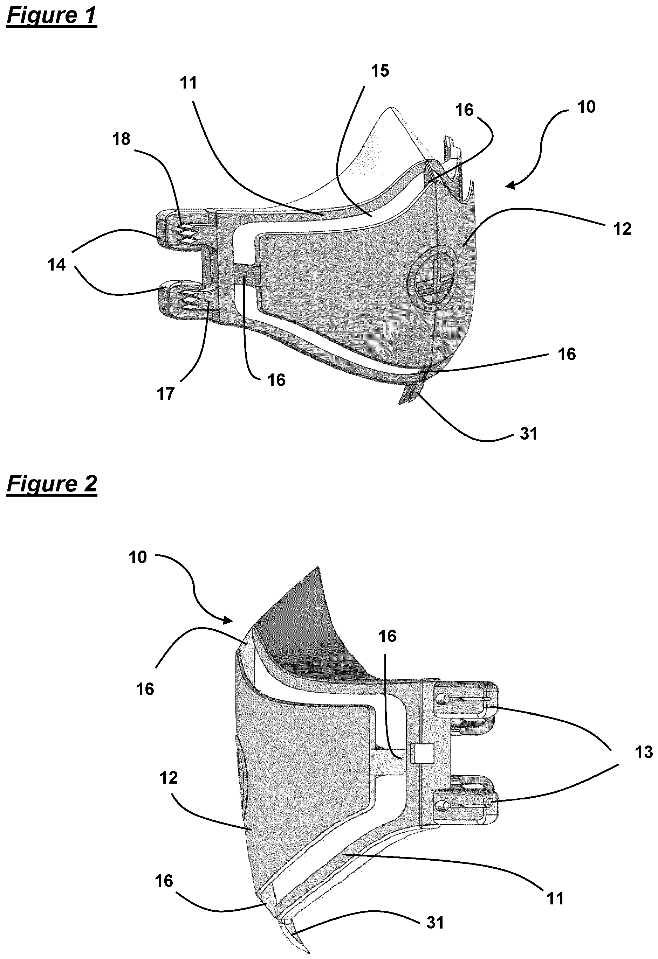

A support frame 10 for use in a filter mask according to the invention is shown in Figures 1 and 2. The support frame 10 has a generally curved shape and comprises an integrally formed rim 11, a shield 12, a pair of first attachment formations 13 and a pair of second attachment formations 14. The shield 12 is substantially the same shape as the rim 11, but is smaller, and is offset from the rim 11 by approximately 1cm. The shield 12 is attached to the rim 11 by four connection members 16 equally spaced about the circumference of the rim, and attached to points close to the edge of the shield. A space 15 between the shield 12 and the rim 11 extends about the periphery of the shield, interrupted only by the connection members 16. A seal supporting member 31 extends downwardly from the centre of the bottom edge of the rim 11. The seal supporting member 31 is an arcuate shaped tab which depends downwardly from the rim 11 and curves towards the rear of the support frame 10. The support frame 10 is an integrally formed component, with all features manufactured in a single injection moulding step.

A pair of first attachment formations 13 outwardly extend from a first side of the rim 11 which, in use, is located near a first ear of the wearer. The first attachment formations 13 take the form of a keyhole shaped slot through which a cord or strap (not shown) may be threaded, and secured with a knot or clamping device. A pair of second attachment formations 14 extend outwardly from a second side of the rim which, in use, is located near the second ear of the wearer. The second attachment formations 14 comprise a slot (not visible) covered by a tab 17. The tab 17 has a series of teeth 18 which provide grip. The cord or strap (not shown) passes through the slot in the attachment formation 14 from the internal side of the support frame to the external side, between the tab 17 and an edge of the slot. The teeth 18 grip the cord and reduce or prevent movement of the cord in the reverse direction.

In use, the cord or strap is secured in the upper first attachment formation 13, passes through the upper second attachment formation 14 from the internal side to the external side, through the lower second attachment formation 14 from the external side to the internal side, and is finally secured in the lower first attachment formation 13. Thus, the strap passes behind the wearer's head twice, with a loop being formed between the pair of second attachment formations 14 on the external side of the support frame. When the wearer applies tension to this loop, eg by pulling the loop, the cord will be pulled through the apertures, past the teeth 18 on the tab 17. The release of this tension eg by the wearer releasing the loop of cord, will allow the teeth 18 to grip the cord, thus preventing the cord from sliding back through the slot unless a significant force is applied. In this way, one hand may be easily used to adjust the mask to fit the wearer's head.

Injection moulding is used to produce the support frame 10, the support frame 10 being an integrally formed unit produced from injection moulded polypropylene in a single step.

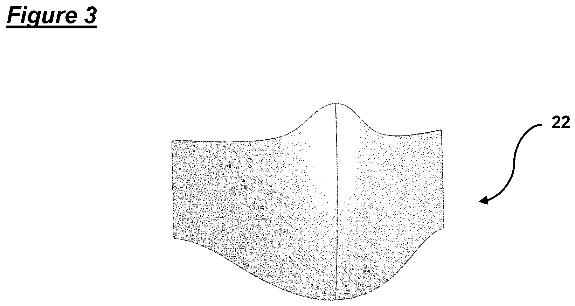

Where the manufacture of the filter mask is automated, the support frame 10 is ejected from the mould onto a vacuum cup on a robot arm. A pneumatic die pushes a sheet of filter material 22 from a roll of filtration material into the support frame 10, the roll of filter material comprising a continuous series of pre-stamped sheets of filter material, heat sealed about their peripheral edges. The sheet of filter material 22 is shown in Figure 3.

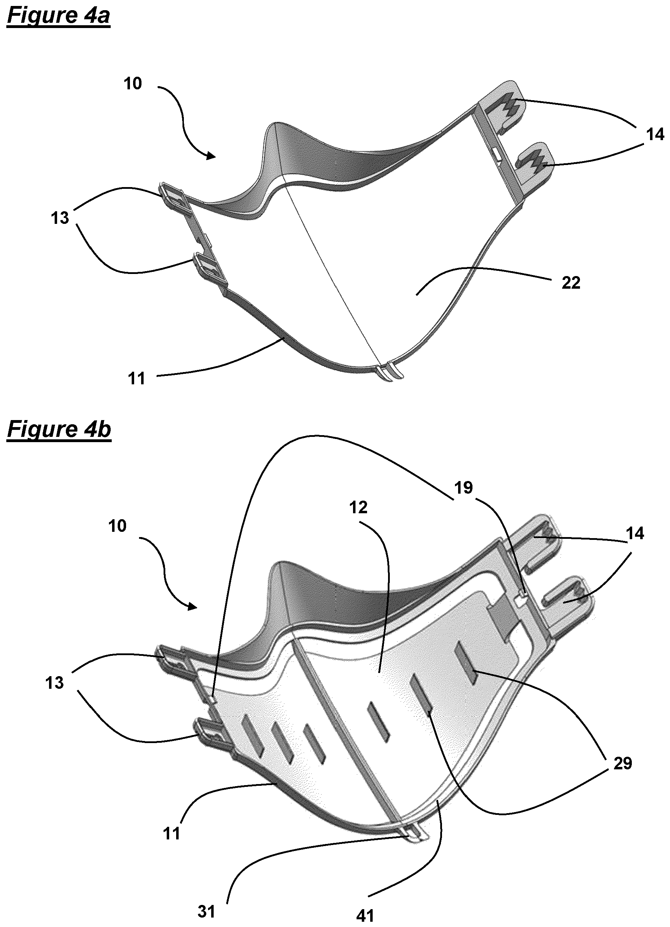

The sheet of filter material is positioned on the internal side of the support frame 10, that is, the side of the support frame 10 which will face the wearer in use. The sheet of filtering material is located on the inwardly extending shoulder, held in place about its periphery by retention tabs 19 at either side of the rim. This support frame/filter material assembly is shown in Figures 4a and 4b.

The support frame/filtration material assembly is transferred into a first half of the mould and mounted to a mould core 30. A second half of the mould is sealed against the first half of the mould, forming the mould cavity. In an injection moulding step, a sealing member 25 is overmoulded to the support frame/filtration material assembly, surrounding the periphery of the sheet of filter material 22 and adhering it to the support frame 10, and forming the sealing member 25, in a single overmoulding step. The filter mask 20 is removed from the mould, eg using a large 20mm pin to unpeel the filter mask 20 from the core 30, and transferred for manual attachment of an elasticated cord and subsequent packaging.

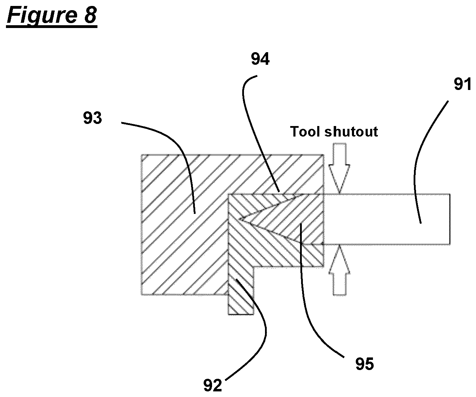

The connection between the filter, sealing member and support frame is shown schematically in Figure 8. The periphery of the filter 91 is placed against a shoulder 94 extending laterally from the rim of the support frame 93, either before or after the support frame is placed in the mould. A thermoplastic elastomer (TPE) 92 is injected into the mould to form the sealing member. The portion of TPE 92 shown in Figure 8 represents only a proximal edge of the sealing member. The TPE 92 is forced by the mould between the peripheral edge of the filter 91 and the rim and shoulder of the support frame 93, 94. The TPE 92 surrounds and impregnates the peripheral edge of the filter 91, the peripheral, impregnated region being denoted 95, adhering it to the rim and shoulder of the support frame 93, 94. Tool shutout, as shown in Figure 8, prevents ingress of TPE 92 into the filter 91 beyond its periphery 95, ensuring that the filtering action of the filter 91 is not inhibited by TPE. As well as aiding adhesion of the support frame 93, 94 to the filter, impregnation of the peripheral edge 95 of the filter 91 with TPE may contribute to the seals at the edge of the filter, formed by heat sealing, preventing moisture or other contaminants from seeping into the structure of the filter.

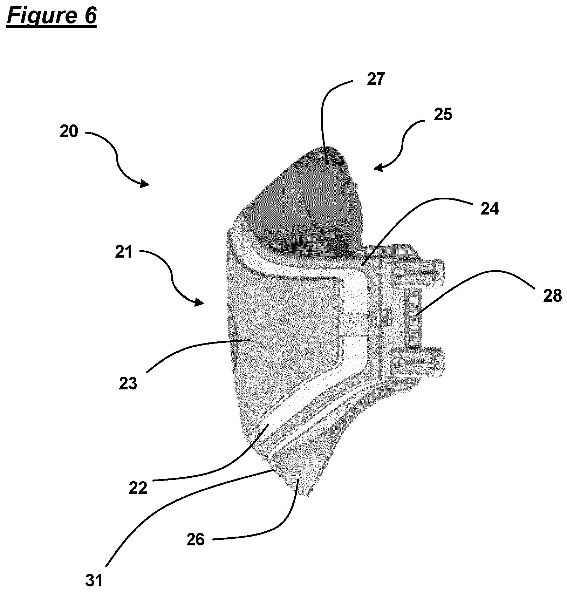

A side view of a filtration mask 20 according to the invention is shown in Figure 6. The support frame 21 is the same as that described in relation to Figures 1 and 2. A sheet of filtering material 22 is positioned behind the support frame 21, and is visible in the gap between the shield 23 and the rim 24, about the periphery of the shield 23. This permits air to easily flow past the shield 23 and through the filtering material 22, while still benefitting from the protection of the shield across the filtering material.

The sealing member 25 is overmoulded onto the support frame 21 and filter material 22, fixing their relative positions. The sealing member 25 comprises a chin engaging portion 26, a nose engaging portion 27 and cheek engaging portions 28. The cheek engaging portions 28 extend laterally across the cheeks, forming a seal against the softer skin on the cheek and improving the overall seal of the mask.

The sealing member 25 is formed of an elastomeric material. It provides a compressible region between the support frame 21 and the wearer's face, enabling the elastomeric material to conform to the contours of the wearer's face and provide a good seal against it.

The downwardly depending seal supporting member 31 abuts the seal in the chin region, reinforcing the seal in this region and enhancing the seal against the wearer's chin.

Figures 4a and 4b show the support frame/filter member assembly, with and without the filter material present respectively. The support frame 10 comprises a rim 11. The rim 11 has an inwardly extending shoulder 41 onto which a pre-cut sheet of filter material 22 is placed. Extending from a first side of the rim are first attachment formations 13, and second attachment formations 14 extend from the second side of the rim. The support frame 10 also comprises a shield 12. A series of vertical ribs 29 across the back of the shield 12 ensure that the spacing between the shield 12 and the filter material 22 is maintained.

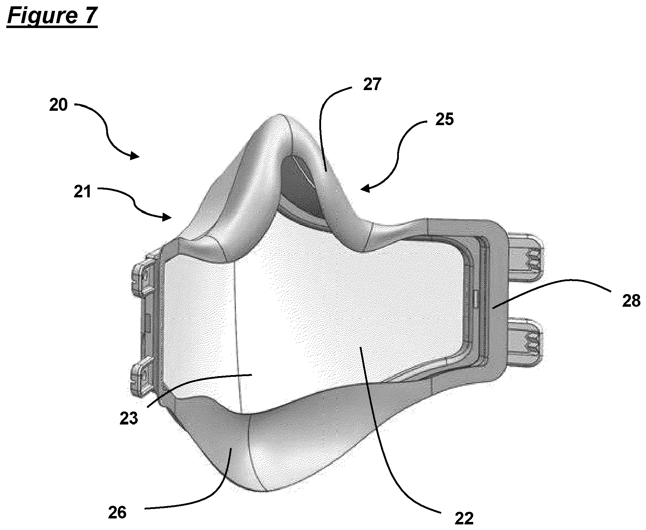

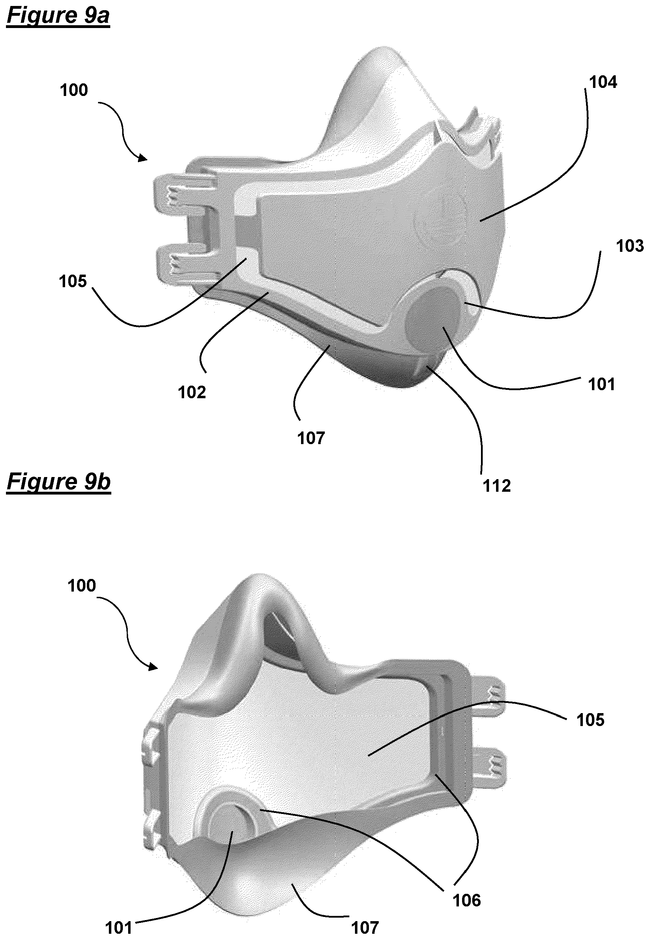

A further embodiment of a filter mask according to the invention can be seen in Figures 9a and 9b, and is generally designated 100. The structure of the mask is substantially the same as that described in relation to the first embodiment depicted in Figures 6 and 7, except that the mask 100 further comprises a mushroom valve 101 (shown in the closed position). In the chin region, the rim 102 of the support frame comprises an upwardly projecting portion 103, and the shape of the shield 104 and filter material 105 corresponds to the shape of the inner periphery of the rim 102 such that they accommodate the upwardly projecting portion 103. The filter material 105 is sealed along the periphery of the rim 102 of the support frame, including the upwardly projecting portion 103, by a surrounding rim of TPE 106. The upwardly projection portion comprises an aperture, with the mushroom valve 101 being formed in the aperture as part of the overmoulding step which also forms the sealing member 107. The mushroom valve 101 permits exhaled air to escape without passing through the filter material 105. The mushroom valve 101 could alternatively be accommodated within the shield 104.

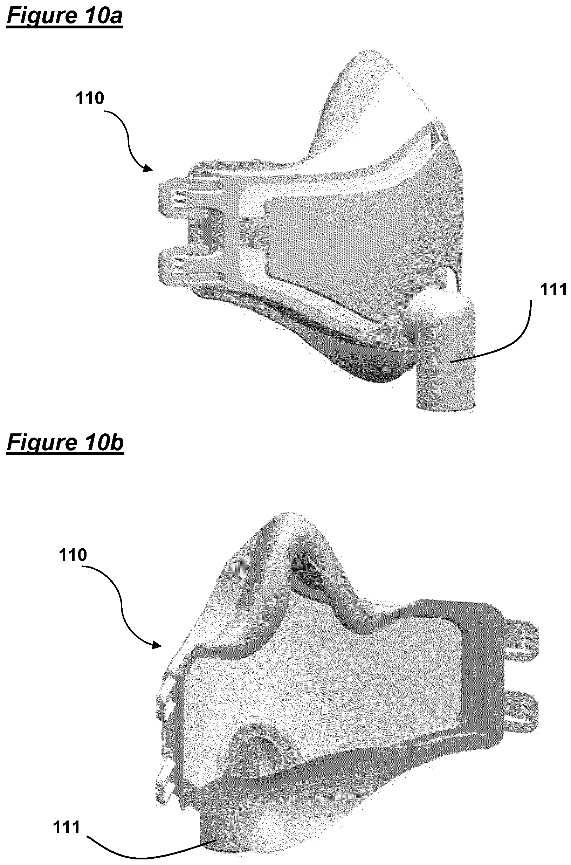

A further embodiment of a filter mask shown in Figures 10a and 10b, which is generally denoted 110, has substantially the same structure is described in the second embodiment in relation to Figures 9a and 9b, but shows an elbow connection 111 in place of the mushroom valve. The elbow connection 111 permits connection of the mask 110 to other medical equipment, such as a nebuliser.

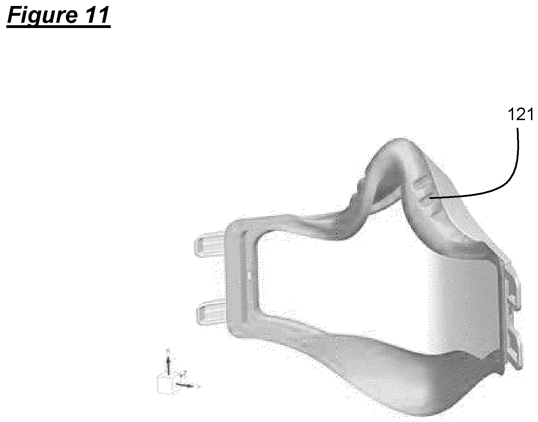

A further embodiment of a filter mask of the invention is shown in Figure 11. The filter mask has all of the features described in relation to Figures 6 and 7, but further comprises a series of corrugations 121 at each side of the nasal portion of the sealing member, to provide reinforcement to the seal. The corrugations 121 extend in a direction perpendicular to the support frame, and extend across a central portion of the seal. The corrugations 121 are integrally formed in the wall of the sealing member, such that they are visible from both the inside and the outside of the sealing member.

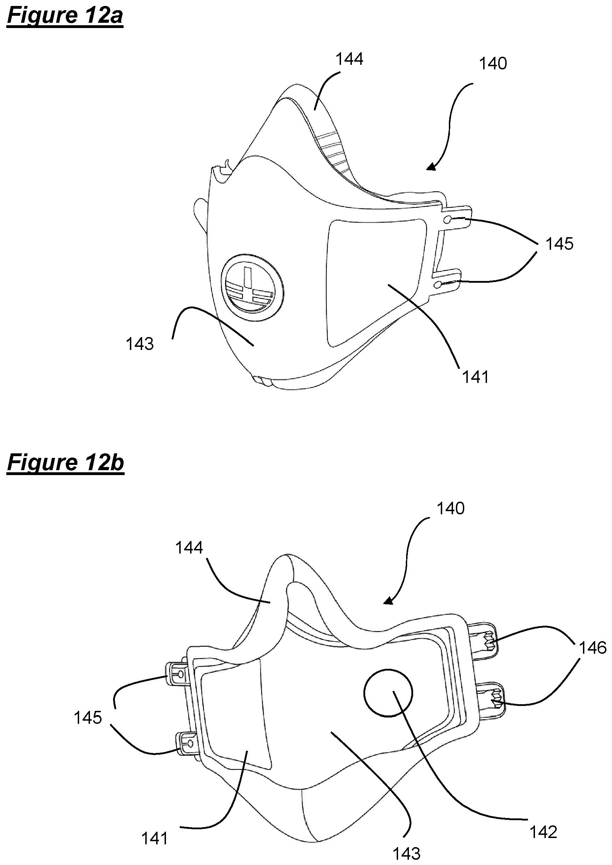

Figures 12a and 12b depict a filter mask 140 comprising a filter 141, a gas inlet 142, a mask body 143, a sealing member 144 depending from a periphery of the mask body 143, and first and second pairs of attachment members 145, 146, which are as described in relation to Figures 4a-b. The gas inlet 142 may be a spigot. An aperture in a first side of the mask body 143 houses the filter 141, while the gas inlet 142 is inserted through or integrally formed with a second side of the mask body 143, such that the filter 141 and gas inlet 142 are located on different sides of the wearer's nose and mouth, in use. During use, oxygen flows from the gas inlet 142 to the filter 141, washing over the wearer's mouth and nose, providing a continuous flow of oxygen and helping to draw away exhaled gases.

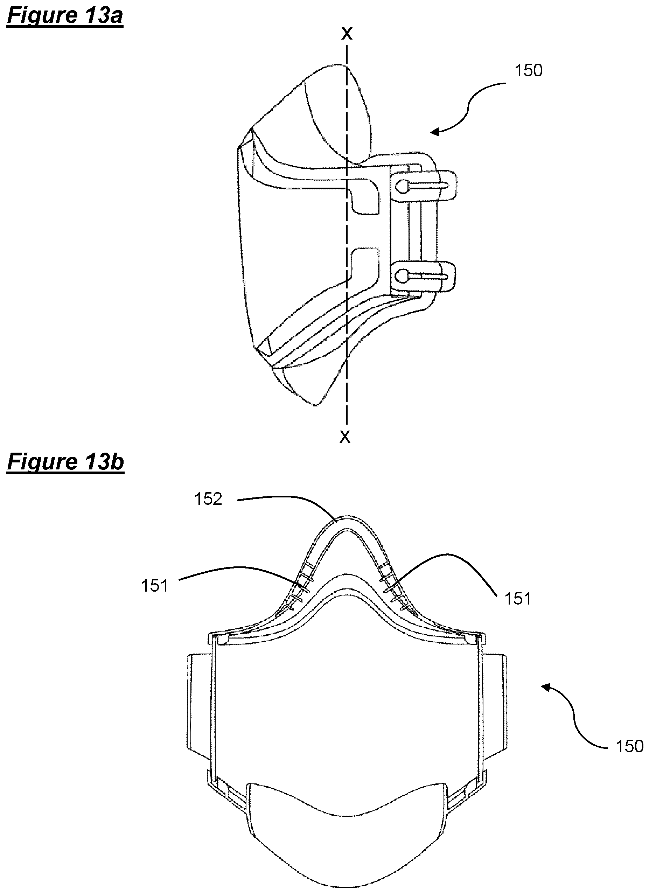

Figure 13b-d show a cross-section of the mask 150 along the line X-X marked on Figure 13a. These drawings show reinforcing members in the nasal region.

Figure 13b shows a series of ribs 151 either side of the nasal region of the sealing member 152. The ribs 151 extend in a direction perpendicular to the mask body, and are located on an internal side wall of the sealing member 152. The ribs provide reinforcement to the sealing member 152, preventing distortion of the sealing member 152 in the nasal region.

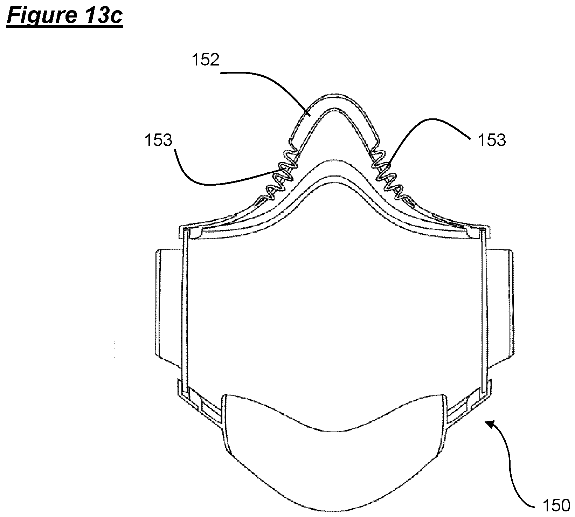

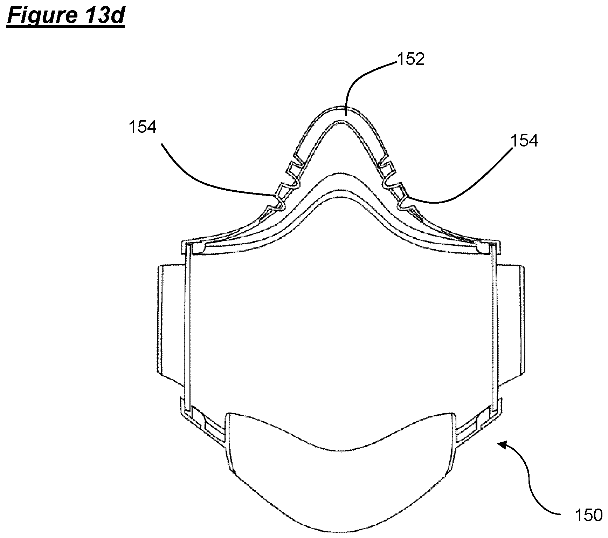

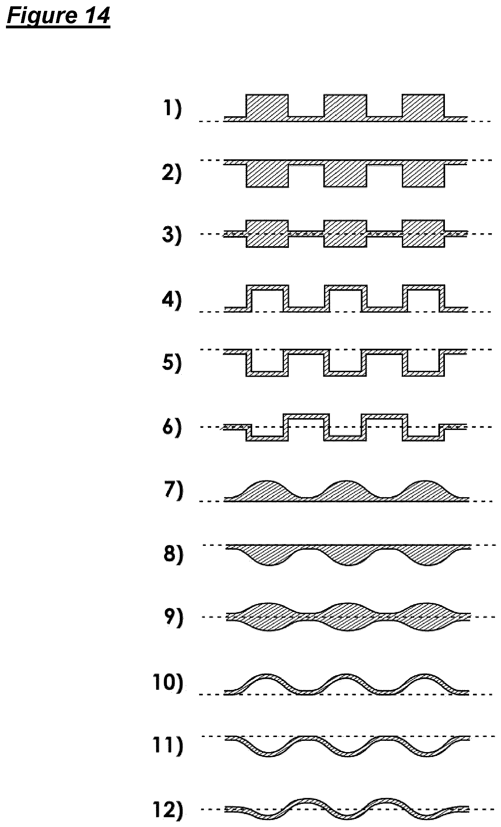

Figures 13c and d show reinforcing members 153, 154 integrally formed within the wall of the sealing member 152. The reinforcing members 153, 154 take the form of corrugations or scallops in the side wall of the sealing member, each corrugation 153 or scallop 154 extending in a direction perpendicular to the mask body. The wall of the sealing member 152 in the region of the reinforcing members 153, 154 retains a uniform thickness. The reinforcing members 153, 154 support the sealing member 152 in the nasal region. Other arrangements of reinforcing members are shown in Figure 14.

## Claims

1. A face mask (20, 100, 110, 140) comprising a sealing member (25) depending from at least a portion of a peripheral edge of the mask body, the sealing member comprising a chin region, a nasal region, and cheek regions intermediate the chin and nasal regions, **characterised in that** the sealing member further comprises both an inwardly and outwardly depending lip portion relative to the peripheral edge of the mask body, wherein the inwardly depending lip portion extends about the chin and nasal regions of the sealing member (26, 27), and the outwardly depending lip portion extends about the chin and cheek regions of the sealing member (26, 28).

2. A face mask (20, 100, 110, 140) as claimed in Claim 1, wherein the outwardly depending lip formation in the chin region (26) has a contact surface that is concave in shape.

3. A face mask (20, 100, 110, 140) as claimed in Claim 2, wherein the outwardly depending lip portion in the chin region (26) is shaped so as to provide a sill or shelf formation beneath the wearer's mandible.

4. A face mask (20, 100, 110, 140) as claimed in Claims 1 to 3, wherein the cheek regions (28) extend laterally across the wearer's cheeks.

5. A face mask (20, 100, 110, 140) as claimed in Claim 4, wherein the cheek portions (28) may extend laterally across the wearer's cheeks between the upper edges of the malar bones and the lower edge of the mandible of the wearer.

6. A face mask (20, 100, 110, 140) as claimed in any of Claims 4 to 5, wherein the distance between a first side edge of the mask body and an opposing side edge of the mask body, measured along the surface of the mask body, may be between 1.2 and 2 times larger than the distance between the top edge of the mask body in the nasal region and the bottom edge of the mask body in the chin region, measured along the surface of the mask body.

7. A face mask (20, 100, 110, 140) as claimed in any one of Claims 4 to 6, wherein the cheek portions (28) each have a first edge that extends from a left- or right- side edge of the nasal portion, and is accommodated over the malar bone of the wearer, and a second edge that extends from a left- or right-side edge of the mouth portion, such that the second edge of the cheek portion is accommodated above the lower edge of the mandible of the wearer, wherein the angle between tangent lines to the first and second edges of each cheek portion decreases with increasing lateral position.

8. A face mask (20, 100, 110, 140) as claimed in Claim 7, wherein the cheek portions (28) each further comprise a side edge which extends between a distal end of the first edge and a distal end of the second edge.

9. A face mask (20, 100, 110, 140) as claimed in Claim 8 where the side edge comprises a substantially linear edge.

10. A face mask (20, 100, 110, 140) as claimed in Claim 8 or 9, wherein the angle between the side edge and the first edge is between 70 and 110 degrees, or between 80 and 100 degrees, or about 90 degrees.

11. A face mask (20, 100, 110, 140) as claimed in any of Claims 7 to 10, wherein the first edge is concave in shape.

12. A face mask (20, 100, 110, 140) as claimed in any preceding claim, which further comprises a port (101, 142), wherein the port is an inlet for respiratory or inhalatory gas.

13. A face mask (20, 100, 110, 140) according to any preceding claim, which further comprises an additive containing silver ions.

## Patentansprüche

1. Gesichtsmaske (20, 100, 110, 140), umfassend ein Dichtungselement (25), das von zumindest einem Abschnitt einer Umfangskante des Maskenkörpers herabhängt, wobei das Dichtungselement einen Kinnbereich, einen Nasenbereich und Wangenbereiche zwischen dem Kinn- und Nasenbereich umfasst, **dadurch gekennzeichnet, dass** das Dichtungselement ferner sowohl einen nach innen als auch nach außen herabhängenden Lippenabschnitt relativ zu der Umfangskante des Maskenkörpers umfasst, wobei sich der nach innen herabhängende Lippenabschnitt um den Kinn- und Nasenbereich des Dichtungselements (26, 27) erstreckt und sich der nach außen herabhängende Lippenabschnitt um den Kinn- und Wangenbereich des Dichtungselements (26, 28) erstreckt.

2. Gesichtsmaske (20, 100, 110, 140) nach Anspruch 1, wobei die nach außen herabhängende Lippenformation in dem Kinnbereich (26) eine Kontaktfläche aufweist, die konkav in der Form ist.

3. Gesichtsmaske (20, 100, 110, 140) nach Anspruch 2, wobei der nach außen herabhängende Lippenabschnitt in dem Kinnbereich (26) dazu geformt ist, eine Schweller- oder Ablageformation unter dem Unterkiefer des Trägers bereitzustellen.

4. Gesichtsmaske (20, 100, 110, 140) nach Anspruch 1 bis 3, wobei sich die Wangenbereiche (28) lateral über die Wangen des Trägers erstrecken.

5. Gesichtsmaske (20, 100, 110, 140) nach Anspruch 4, wobei sich die Wangenabschnitte (28) lateral über die Wangen des Trägers zwischen den Oberkanten der Wangenknochen und der Unterkante des Unterkiefers des Trägers erstrecken können.

6. Gesichtsmaske (20, 100, 110, 140) nach einem der Ansprüche 4 bis 5, wobei der Abstand zwischen einer ersten Seitenkante des Maskenkörpers und einer gegenüberliegenden Seitenkante des Maskenkörpers, gemessen entlang der Oberfläche des Maskenkörpers, zwischen 1,2- und 2-mal größer sein kann als der Abstand zwischen der Oberkante des Maskenkörpers in dem Nasenbereich und der Unterkante des Maskenkörpers in dem Kinnbereich, gemessen entlang der Oberfläche des Maskenkörpers.

7. Gesichtsmaske (20, 100, 110, 140) nach einem der Ansprüche 4 bis 6, wobei die Wangenabschnitte (28) jeweils eine erste Kante, die sich von einer links- oder rechtsseitigen Kante des Nasenabschnittes erstreckt und über dem Wangenknochen des Trägers aufgenommen ist, und eine zweite Kante, die sich von einer links- oder rechtsseitigen Kante des Mundabschnittes erstreckt, aufweisen, sodass die zweite Kante des Wangenabschnittes über der Unterkante des Unterkiefers des Trägers aufgenommen ist, wobei der Winkel zwischen Tangentenlinien zu der ersten und zweiten Kante jedes Wangenabschnittes mit zunehmender lateraler Position abnimmt.

8. Gesichtsmaske (20, 100, 110, 140) nach Anspruch 7, wobei die Wangenabschnitte (28) jeweils ferner eine Seitenkante umfassen, die sich zwischen einem distalen Ende der ersten Kante und einem distalen Ende der zweiten Kante erstreckt.

9. Gesichtsmaske (20, 100, 110, 140) nach Anspruch 8, wobei die Seitenkante eine im Wesentlichen lineare Kante umfasst.

10. Gesichtsmaske (20, 100, 110, 140) nach Anspruch 8 oder 9, wobei der Winkel zwischen der Seitenkante und der ersten Kante zwischen 70 und 110 Grad oder zwischen 80 und 100 Grad oder etwa 90 Grad ist.

11. Gesichtsmaske (20, 100, 110, 140) nach einem der Ansprüche 7 bis 10, wobei die erste Kante konkav in der Form ist.

12. Gesichtsmaske (20, 100, 110, 140) nach einem vorhergehenden Anspruch, die ferner eine Öffnung (101, 142) umfasst, wobei die Öffnung ein Einlass für Atem- oder Inhalationsgas ist.

13. Gesichtsmaske (20, 100, 110, 140) nach einem vorhergehenden Anspruch, die ferner ein Additiv umfasst, das Silberionen enthält.

## Revendications

1. Masque facial (20, 100, 110, 140) comprenant un élément d'étanchéité (25) dépendant d'au moins une partie d'un bord périphérique du corps de masque, l'élément d'étanchéité comprenant une région de menton, une région nasale et des régions de joue intermédiaires entre les régions de menton et nasale, **caractérisé en ce que** l'élément d'étanchéité comprend en outre une partie de lèvre dépendant à la fois vers l'intérieur et vers l'extérieur par rapport au bord périphérique du corps de masque, dans lequel la partie de lèvre dépendant vers l'intérieur s'étend autour des régions de menton et nasale de l'élément d'étanchéité (26, 27), et la partie de lèvre dépendant vers l'extérieur s'étend autour des régions de menton et de joue de l'élément d'étanchéité (26, 28).

2. Masque facial (20, 100, 110, 140) selon la revendication 1, dans lequel la formation de lèvre dépendant vers l'extérieur dans la région de menton (26) présente une surface de contact qui est de forme concave.

3. Masque facial (20, 100, 110, 140) selon la revendication 2, dans lequel la partie de lèvre dépendant vers l'extérieur dans la région de menton (26) est formée de façon à fournir une formation de rebord ou de plateau sous la mandibule de l'utilisateur.

4. Masque facial (20, 100, 110, 140) selon les revendications 1 à 3, dans lequel les régions de joue (28) s'étendent latéralement sur les joues de l'utilisateur.

5. Masque facial (20, 100, 110, 140) selon la revendication 4, dans lequel les parties de joue (28) peuvent s'étendre latéralement sur les joues de l'utilisateur entre les bords supérieurs des os malaires et le bord inférieur de la mandibule de l'utilisateur.

6. Masque facial (20, 100, 110, 140) selon l'une quelconque des revendications 4 à 5, dans lequel la distance entre un premier bord latéral du corps de masque et un bord latéral opposé du corps de masque, mesurée le long de la surface du corps de masque, peut être entre 1,2 et 2 fois plus grande que la distance entre le bord supérieur du corps de masque dans la région nasale et le bord inférieur du corps de masque dans la région de menton, mesurée le long de la surface du corps de masque.

7. Masque facial (20, 100, 110, 140) selon l'une quelconque des revendications 4 à 6, dans lequel les parties de joue (28) présentent chacune un premier bord qui s'étend à partir d'un bord gauche ou droit de la partie nasale, et est logé par dessus l'os malaire de l'utilisateur, et un second bord qui s'étend à partir d'un bord gauche ou droit de la partie de bouche, de sorte que le second bord de la partie de joue soit logé au-dessus du bord inférieur de la mandibule de l'utilisateur, dans lequel l'angle entre des lignes tangentes aux premier et second bords de chaque partie de joue diminue quand la position latérale augmente.

8. Masque facial (20, 100, 110, 140) selon la revendication 7, dans lequel les parties de joue (28) comprennent en outre chacune un bord latéral qui s'étend entre une extrémité distale du premier bord et une extrémité distale du second bord.

9. Masque facial (20, 100, 110, 140) selon la revendication 8, dans lequel le bord latéral comprend un bord sensiblement linéaire.

10. Masque facial (20, 100, 110, 140) selon la revendication 8 ou 9, dans lequel l'angle entre le bord latéral et le premier bord est compris entre 70 et 110 degrés, ou entre 80 et 100 degrés, ou d'environ 90 degrés.

11. Masque facial (20, 100, 110, 140) selon l'une quelconque des revendications 7 à 10, dans lequel le premier bord est de forme concave.

12. Masque facial (20, 100, 110, 140) selon une quelconque revendication précédente, qui comprend en outre un orifice (101, 142), dans lequel l'orifice est une entrée pour du gaz respiratoire ou inhalatoire.

13. Masque facial (20, 100, 110, 140) selon une quelconque revendication précédente, qui comprend en outre un additif contenant des ions argent.
